(19) Europäisches Patentamt

European Patent Office

Office européen des brevets

(11) **EP 0 675 099 B1**

(12) **FASCICULE DE BREVET EUROPEEN**

(45) Date de publication et mention de la délivrance du brevet:
**28.10.1998 Bulletin 1998/44**

(51) Int. Cl.$^6$: **C07C 43/04**, C07C 41/36, C07C 41/42, C07C 41/06

(21) Numéro de dépôt: **95400615.1**

(22) Date de dépôt: **20.03.1995**

(54) **Procédé de purification de l'éthyl tertio butyl éther combinant un procédé à membrane et une distillation**

Ein Membranverfahren und Destillation kombinierendes Verfahren zur Reinigung von Ethyl-Tert-Butylether

Process for the purification of ethyl tert-butyl ether combining a membrane process and a distillation

(84) Etats contractants désignés:
**BE DE ES GB IT NL**

(30) Priorité: **28.03.1994 FR 9403606**

(43) Date de publication de la demande:
**04.10.1995 Bulletin 1995/40**

(73) Titulaire:
**INSTITUT FRANCAIS DU PETROLE**
**92502 Rueil-Malmaison (FR)**

(72) Inventeur: **Streicher, Christian**
**F-92500 Rueil Malmaison (FR)**

(56) Documents cités:
EP-A- 0 331 846          EP-A- 0 497 680
EP-A- 0 507 076          EP-A- 0 542 596
US-A- 4 774 365          US-A- 5 292 963
US-A- 5 294 344

## Description

L'invention concerne la fabrication de l'éthyl tertio butyl éther (en abrégé ETBE)

## État de l'art

On sait que l'éthyl tertio butyl éther, tout comme le méthyl tertio butyl éther (en abrégé MTBE), peut être utilisé comme additif à haut indice d'octane pour les essences sans plomb ou à teneur en plomb réduite. On peut prévoir d'ajouter l'ETBE aux essences à des concentrations allant par exemple jusqu'à environ 15 % en volume.

Un procédé de fabrication du MTBE consiste à réaliser une réaction d'addition de méthanol sur de l'isobutène, par exemple contenu dans une coupe $C_4$ de vapocraquage ou de craquage catalytique. Après réaction, le méthanol résiduel est en général séparé par distillation azéotropique avec les hydrocarbures en $C_4$, ce qui permet d'obtenir aisément le MTBE avec un degré de pureté convenable pour être additionné aux essences.

La fabrication de l'ETBE peut être réalisée par un procédé analogue, dans lequel l'éthanol remplace le méthanol. Un tel procédé est décrit par exemple dans "l'ETBE, un avenir pour l'éthanol" de A. FORESTIERE, B. TORCK et G. PLUCHE, communication faite à la Conférence sur la Biomasse pour l'Energie et l'Industrie, Lisbonne, 9-13 Octobre 1989 et dans "MTBE/ETBE, an Incentive Flexibility for Refiners" par A. FORESTIERE et coll., communication faite à la "Conférence on Oxygenated Fuels in Europe", Londres, 22-23 Mai 1990.

Cependant, par un tel procédé, contrairement au cas du MTBE, après elimination des hydrocarbures en $C_4$, par distillation azéotropique (débutaniseur), la quasi-totalité de l'éthanol résiduel se retrouve en mélange avec l'ETBE produit en fond du débutaniseur. L'existence d'un azéotrope éthanol-ETBE à 21 % en poids d'éthanol à la pression atmosphérique, bouillant à 66,6°C, rend difficile la séparation de l'ETBE avec un degré de pureté suffisant pour satisfaire les spécifications concernant la teneur en éthanol des essences. Ainsi la teneur en éthanol de l'ETBE doit être généralement comprise entre 0,1 et 10 % en poids. Avantageusement, l'ETBE devra être purifié à moins de 1 % en poids d'éthanol pour être additionnée aux essences.

Ainsi, pour que l'ETBE puisse concurrencer le MTBE comme additif améliorant l'indice d'octane des essences sans plomb, il était particulièrement souhaité de trouver un procédé de séparation économiquement attractif.

Divers procédés de purification de l'ETBE obtenu en fond du débutaniseur ont été proposés.

Ainsi, le brevet FR-B-2683523 propose-t-il un procédé dans lequel le mélange alcools/ETBE, obtenu en fond du débutaniseur, est lavé à l'eau, l'extrait eau/alcools ainsi obtenu est ensuite concentré dans une première colonne de distillation puis déshydraté dans deux autres colonnes de distillation hétéroazéotropique utilisant l'ETBE comme agent azéotropant. Ce procédé est cependant relativement complexe et coûteux puisqu'il nécessite l'emploi de 4 colonnes (1 colonne de lavage, 1 colonne de concentration et 2 colonnes de distillation hétéroazéotropique). Il a de plus l'inconvénient de produire un ETBE saturé en eau, ce qui n'est pas avantageux du point de vue de son utilisation comme additif aux essences.

Un procédé plus simple de séparation de mélanges éthanol/ETBE est proposé dans le brevet FR-B-2672048.

Dans ce procédé on utilise le changement de la composition du mélange azéotropique éthanol/ETBE avec la pression. Le mélange éthanol/ETBE est donc séparé par deux colonnes de distillation opérées sous deux pressions différentes. On obtient ainsi l'ETBE pur en fond de la première colonne opérée sous haute pression et l'éthanol pur en fond de la seconde colonne opérée sous basse pression. Les mélanges azéotropiques obtenus en tête de chaque colonne étant recyclés vers l'autre colonne.

Le brevet FR-B-2673624 décrit, lui, un procédé de séparation de mélanges éthanol/ETBE par distillation hétéroazéotropique utilisant l'eau comme agent azéotropant. Dans ce procédé on utilise également deux colonnes de distillation pouvant être opérées sous deux pressions différentes et produisant respectivement l'éthanol et l'ETBE pur en fond de chacune d'elles, de manière analogue au procédé précédent.

Dans les deux procédés précédemment décrits on est cependant amené à mettre en oeuvre deux colonnes de distillation, ce qui rend lesdits procédés là encore relativement coûteux en investissement ainsi qu'en consommation énergétique. De plus, comme il est mentionné dans le brevet FR-B-2672048, les mélanges éthanol/ETBE obtenus en fond du débutaniseur dans les procédés de synthèse d'ETBE contiennent d'autres impuretés comme par exemple l'alcool butylique tertiaire (en abrégé ABT), le diéthyléther (en abrégé DEE), les hydrocarbures en $C_5$ (en abrégé $C_5$), l'éthyl 2-butyl éther (en abrégé E2BE). Or certaines de ces impuretés (DEE, $C_5$) sortent en tête des colonnes de distillation dans les procédés des brevets FR-B-2672048 et FR-B-2673624. Les produits de tête étant entièrement recyclés dans ces procédés, ces impuretés vont s'accumuler progressivement jusqu'à perturber le bon fonctionnement du procédé et dégrader la qualité de la séparation effectuée, ce qui nécessite l'emploi d'une purge en tête de l'une ou l'autre colonne pour pallier cet inconvénient.

Le brevet US-A-4774365 décrit un procédé combinant l'étape de distillation azéotropique (débutaniseur) avec une étape de pervaporation et permettant d'obtenir l'éther produit exempt d'alcool en fond de la colonne de distillation azéotropique.

Dans une première variante de ce procédé, l'effluent de la section réactionnelle passe sur une membrane de pervaporation sélectivement perméable à l'alcool. Le rétentat ainsi produit par l'étape de pervaporation se trouve donc appauvri en alcool. Il est ensuite distillé dans une colonne produisant en fond l'éther produit et en tête les hydrocarbures présents dans l'effluent de la section réactionnelle. Lorsque l'alcool utilisé pour la synthèse d'éther est le méthanol, comme c'est le cas par exemple pour la production de MTBE ou de tertio-amyl-méthyl-éther (en abrégé TAME), le méthanol résiduel qui n'a pas été extrait lors de l'étape de pervaporation se retrouve en tête de cette colonne de distillation avec les hydrocarbures. Ce procédé permet donc bien de produire ces éthers exempts de méthanol. Cependant, si l'on cherche à utiliser un tel procédé pour purifier l'ETBE, l'éthanol se retrouve, comme on l'a déjà signalé, en majeure partie en fond du débutaniseur avec l'ETBE produit. Un tel procédé ne pourrait donc permettre d'obtenir l'ETBE exempt d'éthanol qu'à condition d'extraire tout l'éthanol présent dans l'effluent de la section réactionnelle au niveau de l'étape de pervaporation. Or, la pervaporation, à l'instar de tous les autres procédés membranaires, ne permet pas seule d'obtenir les produits très purs de manière économique; l'extraction, dans ce cas, des dernières traces d'éthanol dans le rétentat conduirait, même avec une membrane très performante, à la mise en oeuvre de surfaces de membranes économiquement irréalistes. Sous cette variante, le procédé proposé par le brevet US-A-4774365 ne permet donc pas d'obtenir l'ETBE exempt d'éthanol de manière économique.

Dans une autre variante du procédé du même brevet, l'effluent de la section réactionnelle est directement admis dans la colonne de distillation azéotropique. Une fraction liquide est prélevée par un soutirage latéral sur cette colonne, puis envoyée vers une étape de pervaporation qui extrait sélectivement une partie de l'alcool. Le rétentat appauvri en alcool produit par cette étape de pervaporation est ensuite recyclé sur la colonne de distillation azéotropique. Cette variante pourrait permettre d'obtenir l'ETBE en fond du débutaniseur à des teneurs en éthanol inférieures à 1 % en poids, voire inférieures à 0,1 % en poids, à condition de pouvoir disposer d'une membrane susceptible d'extraire sélectivement l'éthanol d'un mélange éthanol/ETBE, hydrocarbures. Cependant, le brevet US-A-4774365 ne mentionne que l'existence de membranes susceptibles d'extraire sélectivement le méthanol et la description du procédé de ce brevet ne mentionne sa mise en oeuvre que pour la purification du MTBE et du TAME.

A ce jour, un petit nombre de publications ont fait état de membranes susceptibles d'extraire sélectivement l'éthanol de mélanges organiques : par exemple les brevets US-A-4547530 et US-A-5066403.

Cependant, les membranes décrites dans ces documents ne présentent pas de performances suffisantes (perméabilité, sélectivité, stabilité thermique) pour pouvoir faire l'objet d'une application industrielle. En outre, ces membranes ne permettent pas d'extraire sélectivement des alcools à plus de trois atomes de carbone. Or, comme on l'a vu précédemment, lors de la synthèse de l'ETBE, il se forme une certaine quantité d'ABT, par addition d'une molécule d'eau sur une molécule d'isobutène. La présence d'eau dans la section réactionnelle est inévitable, celle-ci étant introduite comme impureté des produits alimentant ladite section réactionnelle (coupe C4, éthanol), mais également formée dans ladite section réactionnelle elle-même par la réaction secondaire inévitable d'éthérification de l'éthanol au cours de laquelle deux molécules d'éthanol forment une molécule de DEE et une molécule d'eau. La réaction conduisant à la formation de l'ABT étant équilibrée thermodynamiquement, la séparation de l'ABT de l'ETBE produit en fond du débutaniseur et le recyclage de l'ABT vers la section réactionnelle permettraient donc de limiter la conversion de l'isobutène en ABT et par là-même d'accroître la conversion de l'isobutène en ETBE. Il est donc particulièrement souhaitable de trouver un procédé permettant de séparer simultanément les alcools (éthanol/ABT) de l'ETBE, de manière à pouvoir les recycler vers la section réactionnelle, et ce de manière plus simple et plus économique que par les procédés de distillation décrits dans les brevets français mentionnés plus haut.

**Objet de l'invention**

La présente invention a donc pour objet un procédé de purification de l'ETBE combinant l'étape de distillation azéotropique (débutaniseur). habituellement présente après la section réactionnelle dans les procédés de synthèse d'ETBE, avec une étape de perméation, de manière à obtenir l'ETBE en fond du débutaniseur essentiellement exempt des alcools (éthanol, ABT) présents dans l'effluent de la section réactionnelle. L'ETBE ainsi obtenu peut contenir moins de 1 % poids, voire moins de 0,1% poids d'alcools résiduels.

Le procédé de la présente invention peut s'appliquer aussi bien lorsque l'étape de distillation azéotropique est constituée d'une colonne de distillation conventionnelle (débutaniseur) que lorsqu'il s'agit d'une étape de distillation catalytique ou réactive, telle que celle décrite dans "La distillation réactive: principe, applications et perspectives", par P. MIKITENKO, publié dans *Pétrole et techniques*, n° 329, décembre 1986, p. 34-38.

La présente invention a également pour objet un procédé de synthèse d'ETBE incluant le procédé de purification de l'ETBE de la présente invention, dans lequel les alcools extraits lors de l'étape de perméation sont recyclés vers la section réactionnelle ou éventuellement vers la distillation catalytique ou réactive lorsqu'un tel procédé est utilisé.

Le procédé de la présente invention s'adresse plus particulièrement aux mélanges d'ETBE, d'éthanol et d'hydro-carbures en $C_4$ constituant les effluents des sections réactionnelles des procédés de synthèse d'ETBE.

Ces mélanges contiennent généralement de 0,1 à 20 % poids, préférentiellement de 0,5 à 5 % poids d'éthanol et de 5 à 80 % poids, préférentiellement de 10 à 50 % poids d'ETBE, le reste étant essentiellement constitué d'hydrocarbures à 4 atomes de carbone, $C_4$: n-butane, isobutane, butènes. isobutène n'ayant pas réagi. Ils contiennent également en général. à l'état d'impuretés. jusqu'à 1 % poids d'eau, jusqu'à 1 % poids d'ABT, jusqu'à 1 % poids de DEE. jusqu'à 1 % poids d'éther butyl éthylique secondaire (E2BE). jusqu'à 5 % poids, préférentiellement moins de 1 % poids d'hydrocarbures à 5 atomes de carbone, $C_5$: pentanes et pentènes, et jusqu'à 5 % poids d'hydrocarbures à 3 atomes de carbone, $C_3$: propane, propylène.

## Description de l'invention

Le mélange à séparer est introduit dans le débutaniseur. Le débutaniseur est opéré sous une pression généralement supérieure à la pression atmosphérique, de préférence comprise entre 5 et 15 bar. On recueille en tête dudit débutaniseur un distillat contenant l'essentiel des $C_3$, des $C_4$ et de l'eau du mélange à séparer, une fraction de l'éthanol et des $C_5$ du mélange à séparer et essentiellement exempt des autres composés (ETBE, E2BE, ABT, DEE). On recueille en fond du débutaniseur l'essentiel de l'ETBE et des autres éthers (E2BE, DEE) avec une fraction des $C_5$ et de faibles quantités (moins de 1 % poids, préférentiellement moins de 0,1 % poids) d'alcools (éthanol, ABT).

On soutire latéralement dudit débutaniseur au moins une phase vapeur, liquide ou mixte, que l'on envoie dans une zone de perméation.

Par zone de perméation, on entend une zone de pervaporation lorsqu'une phase liquide est mise en contact avec la face amont de la membrane ou une zone de perméation de vapeur lorsqu'il s'agit d'une phase gazeuse ou mixte.

Selon une caractéristique du procédé, on effectue un soutirage latéral d'au moins une phase au niveau d'au moins un plateau du débutaniseur dans lequel la concentration en éthanol est sensiblement maximale.

Selon une autre caractéristique du procédé, la zone de perméation est équipée d'une membrane composite habituellement constituée d'une couche sélective déposée sur au moins une couche support poreuse. Alors que la (ou les) couche(s) support(s) peut (peuvent) être constituée(s) de tout type de matériaux organiques ou inorganiques habituellement utilisés pour réaliser des supports de membranes, la couche sélective est, elle, caractéristique du procédé de la présente invention et est constituée d'un film dense d'un homo-, co-, ou terpolymère. L'homopolymère utilisé est le poly N,N-diméthylaminoéthyl-méthacrylate (en abrégé DMAEMA), le copolymère peut être constitué de DMAEMA et de N-vinylcaprolactame (en abrégé NVCL) ou de DMAEMA et de N-vinylpyrrolidone (en abrégé NVP) et le terpolymère est constitué de DMAEMA, NVCL et de NVP. Le groupement amine du DMAEMA est préférentiellement quaternisé en un groupement ammonium quaternaire. Les réactifs de quaternisation préférés sont le diméthyl- et le diéthylsulfate, ainsi que le monochloro-, monobromo- ou monoiodo-méthane ou éthane. Après dépôt par enduction de la couche sélective sur la (ou les) couche(s) support(s), la membrane formée est traitée thermiquement à des températures de 100 à 200°C pendant des durées de 1 à 60 minutes, de manière à rendre la couche sélective insoluble dans l'eau, les alcools et les solvants organiques. Cette couche sélective offre l'avantage d'être sélectivement perméable aux alcools, non seulement à l'éthanol, mais également à d'autres alcools et notamment à l'ABT. On peut donc ainsi extraire ces composés de la (ou des) phase(s) soutirée(s).

Selon une autre caractéristique du procédé, la (ou les) phase(s) soutirée(s) du débutaniseur est (sont) admise(s) dans la zone de perméation à une température et une pression qui peuvent être supérieures ou inférieures à celles auxquelles elle(s) se trouve(nt) lors du soutirage. Les éventuels changements de température et de pression peuvent être effectués au moyen des équipements appropriés (pompes, compresseurs, vannes de détente, échangeurs).

Selon une autre caractéristique du procédé, le rétentat appauvri en alcools n'ayant pas traversé la membrane peut être recyclé dans la zone de distillation. Ce rétentat peut être obtenu après la zone de perméation en phase liquide. gazeuse ou mixte. Il doit, pour être recyclé dans la zone de distillation être porté à une pression au moins égale à celle dans ladite zone de distillation, au moyen des équipements appropriés (pompes, compresseurs). Il peut également être porté à une température supérieure ou inférieure à celle à laquelle il est recueilli après la zone de perméation et ce au moyen des équipements appropriés (échangeurs). La (ou les) phase(s) ainsi recyclée(s) dans la zone de distillation l'est (le sont) avantageusement à un (des) plateau(x) dont la composition est sensiblement la même que celle de ladite (desdites) phase(s) recyclée(s).

Enfin, on recueille en aval de la membrane de perméation un perméat constitué majoritairement d'alcools (éthanol, ABT), qui peut avantageusement être recyclé vers la section réactionnelle.

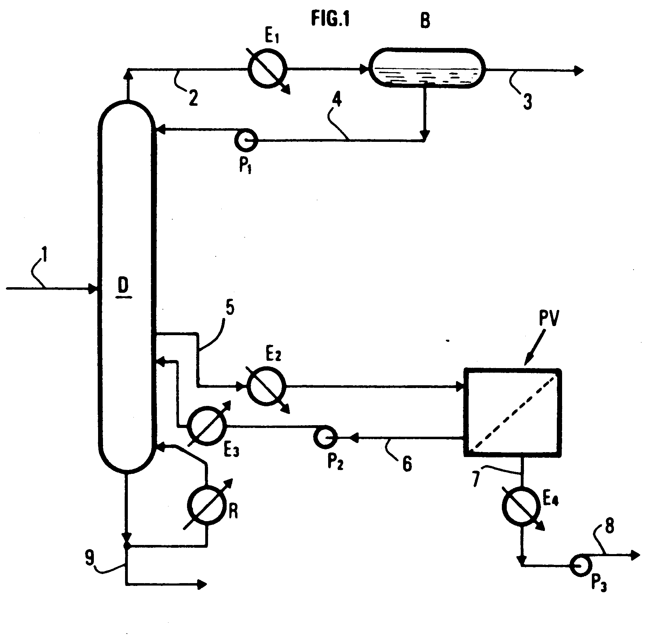

On décrit un mode avantageux de réalisation de l'invention en relation avec la figure 1.

Le mélange à séparer est envoyé par la ligne 1 dans la colonne de distillation D (débutaniseur). Cette colonne opère généralement sous une pression p supérieure à 1 bar, préférentiellement comprise entre 5 et 15 bar. Elle est chauffée par le rebouilleur R. La température de fond est en général comprise entre 70 et 200°C. La température de tête est en général comprise entre 30 et 100°C. Le mélange à séparer est introduit dans le débutaniseur D préférentiellement au point de bulle à la pression considérée au plateau ayant la composition la plus voisine possible de celle dudit mélange, par exemple dans la partie médiane de la colonne dans le cas de mélanges contenant de 10 à 50 % en poids d'ETBE.

Le produit sortant en fond du débutaniseur par la ligne 9 est constitué de l'ETBE purifié. La quasi-totalité des éthers, ETBE mais également E2BE et DEE, présents dans le mélange à séparer sont ainsi récupérés essentiellement exempts de $C_3$, $C_4$ et d'eau, à une teneur en $C_5$ fonction de celle du mélange à séparer, usuellement comprise entre 0,1 et 1 % en poids et à des teneurs résiduelles en alcools (éthanol et ABT), fonctions des conditions opératoires du procédé, inférieures à 1% en poids et pouvant même être inférieures à 0,1 % en poids.

En tête du débutaniseur on récupère par la ligne 2 un distillat en phase vapeur constitué d'hydrocarbures $C_3$, $C_4$ et $C_5$, contenant une proportion variable d'éthanol, généralement comprise entre 1 et 5 % poids, de l'eau à une teneur généralement inférieure à 1 % poids et essentiellement exempt d'ABT et d'éthers (ETBE, E2BE, DEE).

Cette phase vapeur est entièrement condensée et éventuellement sous-refroidie dans l'échangeur E1 puis admise dans le ballon décanteur B. Du ballon B on recueille par la ligne 3 un distillat liquide de même composition et contenant la quasi-totalité de l'eau et des hydrocarbures $C_3$, $C_4$ du mélange à séparer. Le reste du liquide recueilli au niveau du ballon B est envoyé par la ligne 4, grâce à la pompe P1, en reflux en tête du débutaniseur D.

Une fraction préférentiellement liquide est prélevée par la ligne 5 sur un soutirage latéral du débutaniseur D. Ce soutirage latéral est préférentiellement placé au niveau du plateau où la concentration en éthanol dans la phase liquide est maximale, en général à un plateau inférieur à celui de l'alimentation du débutaniseur D par le mélange à séparer. Le débit massique de la fraction ainsi prélevée est un paramètre opératoire important du procédé. Il doit être ajusté en fonction des autres paramètres opératoires et de la teneur en alcools (éthanol, ABT) résiduels requise dans l'ETBE recueilli en fond du débutaniseur D.

La fraction liquide ainsi prélevée latéralement est portée dans l'échangeur E2 à une température généralement comprise entre 50 et 120°C, préférentiellement comprise entre 80 et 100°C, puis admise dans la zone de pervaporation PV.

Ladite zone de pervaporation PV est équipée d'une membrane comportant une couche dense sélective constituée d'un film d'homo-, co-ou terpolymère, l'homopolymère étant le DMAEMA, les copolymères pouvant être constitués de DMAEMA et de NVCL ou de DMAEMA et de NVP, le terpolymère étant constitué de DMAEMA, NVCL et de NVP, le matériau préférentiellement utilisé étant un copolymère de NVP et de DMAEMA, le groupement amine du DMAEMA étant préférentiellement quaternisé en ammonium quaternaire par réaction avec le diméthylsulfate ou le diéthylsulfate ou le monochloro-, ou monobromo-, ou monoiodométhane ou éthane.

Si l'on désigne par x,y et z les fractions molaires respectives de DMAEMA, de NVP et de NVCL, normalisées de sorte qu'on ait: x+y+z=1 ; x peut alors être compris entre 1 (homopolymère) et 0,1; y et z étant comprises chacunes entre 0 et 0,9. Pour les copolymères (y=0 ou z=0), les valeurs de x comprises entre 0,2 et 0,8 sont préférées et tout particulièrement celles entre 0,4 et 0,6. Pour les terpolymères les valeurs de x,y et z comprises chacunes entre 0,2 et 0,5 sont préférées.

La masse moléculaire moyenne des homo-, co- ou terpolymères peut être comprise entre 50 000 et 5 000 000 Dalton, préférentiellement entre 100000 et 1000000 Dalton.

Ladite couche dense sélective est formée par étalement sur un support d'une solution du polymère dans l'eau, l'éthanol ou leurs mélanges. Ladite solution peut contenir de 2 à 50 % en poids de polymère, préférentiellement de 4 à 20 % en poids. Après évaporation du solvant de ladite solution étalée, à des températures inférieures à 100°C, la membrane obtenue est traitée thermiquement à des températures comprises entre 100 et 200°C, préférentiellement comprises entre 120 et 160°C, pendant des durées comprises entre 1 et 60 minutes. Ledit traitement thermique est préférentiellement effectué en deux étapes, une première étape, par exemple d'une durée de 1 à 30 minutes, à des températures comprises entre 100 et 120°C, par exemple 100°C, la seconde étape, par exemple d'une durée de 1 à 30 minutes, à des températures comprises entre 110°C et 200°C, par exemple 140°C.

L'épaisseur de ladite couche dense sélective ainsi obtenue dépend de la viscosité de ladite solution de polymère et de la méthode d'étalement utilisée. Elle sera préférentiellemnt comprise entre 0,5 et 20 $\mu$m, plus préférentiellement comprise entre 1 et 5 $\mu$m.

Bien que la membrane utilisée dans ladite zone de pervaporation PV puisse être constituée de ladite couche dense sélective seule, elle est préférentiellement constituée d'une structure composite dans laquelle ladite couche dense sélective est formée par enduction de ladite solution de polymère à la surface d'une structure poreuse préférentiellement constituée :

- d'une base préférentiellement constituée d'une feuille tissée ou non tissée faite à partir de fibres de matériaux organiques tels que polyéthylènes, polypropylènes, polyamides, polyesters, polyphénylènesulfures et autres matériaux apparentés, ou à partir de fibres de matériaux inorganiques tels que carbone ou verre ; ladite base peut également être constituée d'un corps en céramique poreuse;

- d'une couche support poreuse, sur laquelle est déposée ladite couche dense sélective, ayant une structure asymétrique, déposée sur ladite base et préférentiellement constituée de matériaux organiques tels que polysulfones, polyoléfines, polyacrylonitriles, polyamides et autres matériaux apparentés; ladite couche support poreuse peut également être constituée de matériaux inorganiques tels que des céramiques par exemple ;

L'unité de pervaporation PV produit par la ligne 6 un rétentat appauvri en alcools (éthanol, ABT). Ledit rétentat est porté par la pompe P2 à une pression suffisante pour pouvoir être retourné au débutaniseur D. Ledit rétentat peut être réchauffé et éventuellement au moins partiellement vaporisé dans l'échangeur E3 avant d'être retourné au débutaniseur D. Ledit rétentat est préférentiellement recyclé au débutaniseur D au point de bulle à la pression considérée, au plateau dont le liquide a la composition la plus voisine de celle dudit rétentat, généralement à un plateau inférieur à celui auquel on effectue le soutirage latéral.

Dans le cas où ledit rétentat est au moins partiellement vaporisé avant d'être recyclé au débutaniseur D il peut être avantageux, après séparation dans un ballon séparateur de la fraction vaporisée et de la fraction liquide dudit rétentat, de recycler ladite fraction vaporisée au débutaniseur D à une plateau supérieur à celui auquel est admise ladite fraction liquide et éventuellement supérieur à celui auquel est effectué le soutirage latéral.

L'unité de pervaporation PV produit par la ligne 7 un perméat en phase vapeur à une pression généralement inférieure à la pression atmosphérique, préférentiellement comprise entre 10 et 500 mbar, majoritairement constitué d'alcools (éthanol, ABT), la composition de ce perméat est fonction des conditions opératoires du procédé (débit et composition de la phase soutirée latéralement du débutaniseur D, surface de membrane mise en oeuvre). Ledit perméat contient le plus fréquemment plus de 90 % en poids d'alcools. Il est ensuite condensé dans l'échangeur E4, puis porté à une pression supérieure à la pression atmosphérique, préférentiellement suffisante pour qu'il puisse être recyclé vers la section réactionnelle, par la pompe P3 et recueilli par la ligne 8 d'où il sera préférentiellement recyclé vers la section réactionnelle.

## Avantages du procédé

Le procédé de la présente invention a pour principal avantage sa très grande simplicité puisqu'il permet d'obtenir l'ETBE purifié avec une seule unité de perméation en plus du débutaniseur présent dans tous les procédés précédemment décrits de synthèse d'ETBE, alors que tous les autres procédés précédemment décrits de purification d'ETBE nécessitent l'emploi d'au moins deux colonnes en plus dudit débutaniseur. Le procédé de la présente invention s'avère donc particulièrement économe en investissement.

En outre, l'emploi préféré, en plus du débutaniseur, de la perméation, qui est un procédé de séparation nettement moins consommateur d'énergie que la distillation, rend le procédé de la présente invention particulièrement économe en énergie.

Le procédé de la présente invention a en outre l'avantage de ne pas permettre l'accumulation des impuretés du mélange éthanol/ETBE/$C_4$ à séparer (ABT, $C_3$, $C_5$, eau, DEE, E2BE), celles-ci étant éliminées avec les différents produits issus du procédé (mélange $C_4$/éthanol en tête du débutaniseur, ETBE en fond du débutaniseur et perméat de l'unité de perméation PV). Le procédé de la présente invention ne nécessite de ce fait pas de purge, contrairement aux procédés décrits dans les brevets FR-B-2672048 et FR-B-2673624.

Enfin, l'utilisation dans le procédé de la présente invention d'une membrane spécifique, sélectivement perméable non seulement à l'éthanol mais également à l'ABT, permet de recycler ces produits vers la section réactionnelle et, du fait du recyclage d'une majeure partie de l'ABT formé au niveau de ladite section réactionnelle, de réduire considérablement la conversion de l'isobutène en ABT, comme mentionné précédemment, ce qui accroît d'autant la conversion de l'isobutène en ETBE.

Une variante du procédé de l'invention consiste à remplacer le débutaniseur D par une colonne de distillation catalytique ou réactive D', ce qui permet d'augmenter les taux de conversion de l'isobutène et/ou de réduire l'excès d'éthanol employé. Les effluents de la colonne D' auront des compositions analogues à celles de ceux du débutaniseur D avec simplement des teneurs en éthanol généralement réduites.

Le procédé de purification d'ETBE à partir d'un mélange ETBE/éthanol/ hydrocarbures, tel qu'il a été décrit ci-dessus, peut aisément être intégré dans un procédé de synthèse d'ETBE à partir d'éthanol et d'isobutène contenu dans une coupe $C_4$ pouvant par exemple provenir d'une unité de vapocraquage, de craquage catalytique ou de déshydrogénation des butanes.

Un tel procédé de synthèse d'ETBE est décrit ci-dessous en liaison avec la figure 2. Sur cette figure 2, on n'a pas représenté les divers équipements (pompes, échangeurs de chaleur, vannes de détente, ballons,...) nécessaires au bon fonctionnement des différentes étapes du procédé.

La coupe $C_4$ contenant l'isobutène ainsi que des quantités mineures de $C_3$ et de $C_5$ est amenée en phase liquide par la ligne 20. L'éthanol d'appoint nécessaire à la réaction est amené en phase liquide par la ligne 21. L'azéotrope eau/éthanol produit par la section LD de lavage à l'eau du raffinat $C_4$ du débutaniseur D (ou D') est recyclé en phase liquide par la ligne 24. Le perméat condensé produit par la zone de perméation PV est recyclé en phase liquide par la ligne 8. Ces divers fluides sont mélangés puis admis dans la section réactionnelle R par la ligne 22.

La section réactionnelle R produit par la ligne 1 un mélange constitué de l'ETBE produit, des hydrocarbures $C_4$ non réactifs et non réagis, de l'excès d'éthanol n'ayant pas réagi ainsi que des diverses impuretés déjà mentionnées (ABT,

eau, $C_3$, $C_5$, DEE, E2BE). Ce mélange alimente le débutaniseur D.

Du débutaniseur D (ou D') on recueille en fond par ligne 9 l'ETBE purifié, avec les autres éthers à l'état de traces (DEE, E2BE) et en tête les hydrocarbures, l'eau et une proportion variable, généralement de 1 à 5 % en poids d'éthanol. Ce raffinat, une fois liquéfié, est admis par la ligne 3 dans la section de lavage à l'eau LD.

Cette section de lavage à l'eau LD est en fait constituée d'une étape de lavage à l'eau proprement dit produisant par la ligne 23 les hydrocarbures essentiellement exempts d'éthanol, ainsi qu'un mélange eau/éthanol, et d'une étape de distillation dudit mélange produisant en fond de l'eau recyclée vers le lavage à l'eau et en tête un mélange azéotropique eau/éthanol contenant environ 5 % en poids d'eau qui est recyclé par la ligne 24 vers la section réactionnelle R.

Une phase est soutirée latéralement du débutaniseur D (ou D') et amenée par la ligne 5 dans la zone de perméation PV.

Cette zone de perméation PV produit un rétentat appauvri en alcools (éthanol, ABT) qui est recyclé par la ligne 6 vers le débutaniseur D (ou D'), et un perméat majoritairement constitué d'alcools (éthanol, ABT) recyclé après condensation par la ligne 8 vers la section réactionnelle R.

Dans le cas où le débutaniseur D est remplacé par une colonne de distillation catalytique ou réactive D', il peut être avantageux de recycler au moins partiellement le perméat majoritairement constitué d'alcools, produit par la zone de perméation PV, par la ligne 25 vers ladite colonne de distillation catalytique ou réactive D'.

Il peut alors de même être avantageux de recycler au moins partiellement le mélange azéotropique éthanol/eau produit par la section de lavage à l'eau LD, par la ligne 26 vers la zone de distillation catalytique ou réactive D'.

Les exemples suivants illustrent l'invention.

## Exemple 1

Une solution de copolymère de NVP et de DMAEMA est déposée sur une couche poreuse de polyacrylonitrile (en abrégé PAN) elle-même supportée par une feuille de polyester non tissé. Le rapport molaire des monomères NVP et DMAEMA dans le copolymère est de 1:1. La solution de copolymère est constituée d'un mélange à 10 % en poids de copolymère dans l'eau. Après dépôt sur le support poreux la solution est évaporée pendant 2 min. à 90 °C. La membrane ainsi obtenue est ensuite traitée thermiquement pendant 30 min. à 125 °C.

On effectue avec cette membrane un test de pervaporation avec un mélange constitué de 25 % en poids d'éthanol et de 75 % en poids d'ETBE, à une température de 95 °C. La pression en aval de la membrane est de 20 mbar. Le perméat est condensé à une température de 0 °C. On obtient alors un flux de perméat de 6,2 $kg.h^{-1}.m^{-2}$. La teneur en éthanol du perméat est de 94,7 % en poids.

## Exemple 2

Une solution équipondérale d'eau et d'éthanol contenant 7 % en poids de DMAEMA est déposée sur un support identique à celui utilisé dans l'exemple 1. Après évaporation de la solution pendant 2 min. à 80 °C, la membrane obtenue est traitée thermiquement pendant 15 min. à 150 °C. L'épaisseur de la couche sélective de DMAEMA est alors de 3 $\mu$m.

On effectue avec cette membrane un test de pervaporation avec un mélange contenant 20 % en poids d'éthanol et 80 % en poids d'ETBE à 60 °C. La pression en aval de la membrane est de 6 mbar et le perméat est condensé dans un piège plongé dans un mélange d'éthanol et de glace carbonique. Le flux de perméat ainsi obtenu est de 0,92 $kg.h^{-1}.m^{-2}$, sa teneur en éthanol est de 98,2 % en poids.

## Exemple 3

Une solution de copolymère de NVP et de DMAEMA est déposée sur une couche poreuse de polyfluorovinylidène (en abrégé PVDF) elle-même déposée sur une feuille de polyphénylènesulfure (en abrégé PPS) non tissé. Le groupement amine du DMAEMA est quaternisé par le diéthylsulfate. Le rapport molaire du NVP par rapport au DMAEMA quaternisé est de 3:2. La solution est constituée de 4,5 % en poids de copolymère dans de l'eau. Après évaporation de la solution pendant 2 min. à 80 °C, la membrane obtenue est traitée thermiquement pendant 10 min. à 115 °C puis pendant 5 min. à 150 °C.

On effectue avec cette membrane un test de pervaporation avec un mélange constitué de 20 % en poids d'éthanol et de 80 % en poids de toluène, à 80 °C. La pression en aval de la membrane est de 10 mbar. Le perméat est condensé à une température de 0 °C. On obtient alors un flux de perméat de 2,5 $kg.h^{-1}.m^{-2}$, le perméat contenant 98,8 % en poids d'éthanol.

**Exemple 4**

Une solution à 5 % en poids dans l'eau d'un terpolymère de NVCL, NVP et de DMAEMA est déposée sur un support identique à celui de l'exemple 1. Le groupement amine du DMAEMA est quaternisé par le monochlorométhane. La composition molaire du terpolymère en monomères de NVCL, NVP et DMAEMA respectivement, est de 0,3:0,3:0,4. Après évaporation de la solution pendant 30 s à 95 °C, la membrane obtenue est traitée thermiquement pendant 10 min. à 120 °C puis pendant 8 min. à 145 °C.

On effectue avec cette membrane un test de pervaporation avec un mélange constitué de 20 % en poids d'éthanol et de 80 % en poids de n-heptane, à 50 °C. La pression en aval de la membrane est de 5 mbar. Le perméat est condensé dans un piège plongé dans un mélange d'éthanol et de glace carbonique. On obtient alors un flux de perméat de 1,8 kg.h$^{-1}$.m$^{-2}$, le perméat contenant 99,2 % en poids d'éthanol.

On effectue ensuite avec une membrane préparée de la même manière un test de pervaporation avec un mélange constitué de 5 % en poids de méthanol et de 95 % en poids de n-heptane, à 55 °C. La pression en aval de la membrane est de 5 mbar. Le perméat est condensé dans un piège plongé dans un mélange d'éthanol et de glace carbonique. On obtient alors un flux de 2,3 kg.h$^{-1}$.m$^{-2}$, le perméat contenant 99,8 % en poids de méthanol.

On effectue enfin, toujours avec une membrane préparée de la même manière, un test de pervaporation avec un mélange constitué de 10 % en poids de 2-propanol et de 90 % en poids de n-heptane, dans les mêmes conditions. On obtient alors un flux de 0,8 kg.h$^{-1}$.m$^{-2}$, le perméat contenant 92,7 % en poids de 2-propanol.

**Exemple 5**

On réalise une membrane suivant la procédure décrite à l'exemple 1. On effectue avec cette membrane un test de pervaporation avec un mélange constitué de 0,1 % en poids d'eau ; 15,6 % en poids d'éthanol ; 1,0 % en poids de DEE ; 5,3 % en poids d'ABT et 78,0 % en poids d'ETBE, à une température de 48 °C. La pression en aval de la membrane est de 8 mbar. Le perméat est condensé dans un piège plongé dans l'azote liquide. On obtient alors un flux de perméat de 0,12 kg.h$^{-1}$.m$^{-2}$ ayant la composition suivante : eau : 4,5 % en poids, éthanol : 90,8 % en poids, DEE : 0,3 % en poids, ABT : 2,3 % en poids et ETBE : 2,1 % en poids. La sélectivité $\alpha_{i/j}$ de la membrane pour le composé i par rapport au composé j est calculée par la formule :

$$\alpha_{i/j} = \frac{c'_i}{c_i} / \frac{c'_j}{c_j}$$

où $c'_i$ et $c'_j$ désignent les concentrations massiques respectives des composés i et j dans le perméat, $c_i$ et $c_j$ désignent les concentrations massiques respectives des composés i et j dans le mélange en amont de la membrane. Avec cette formule, l'expérience de pervaporation ainsi réalisée permet de calculer :

$\alpha_{eau/ETBE}$ = 1671
$\alpha_{éthanol/ETBE}$ = 216
$\alpha_{ABT/ETBE}$ = 16
$\alpha_{DEE/ETBE}$ = 11

Cet exemple illustre le fait que la membrane considérée s'avère sélectivement perméable non seulement à l'éthanol mais également aux autres composés oxygénés et en particulier à l'ABT en présence de mélanges de ces produits avec l'ETBE.

**Exemple 6**

On a réalisé la séparation d'un mélange C$_4$/éthanol/ETBE représentatif d'un effluent de section réactionnelle d'un procédé d'éthérification de manière à obtenir la coupe C4 en tête du débutaniseur D à moins de 1 ppm en poids d'ETBE et l'ETBE en fond du débutaniseur à moins de 0,1 % en poids d'alcools résiduels.

La composition et le débit du mélange à séparer, ALIMENTATION, sont donnés dans le tableau 1.

Le débutaniseur D est constitué d'une colonne en acier inoxydable d'un diamètre intérieur de 100 mm, comportant 70 plateaux perforés à déversoir espacés de 5 cm.

Il est isolé thermiquement de manière à éviter toutes les déperditions de calories et muni d'un bouilleur chauffé électriquement R, d'un condenseur à eau froide E1 et d'un ballon de reflux B.

Les plateaux du débutaniseur sont numérotés par ordre croissant à partir de 1 du haut vers le bas.

Le débutaniseur D est opéré sous une pression, mesurée au ballon de reflux B, de 8,0 bar. La température de la colonne D s'étage entre 161,2 °C en fond et 67,5 °C en tête. Le distillat obtenu en tête du débutaniseur D est condensé puis sous-refroidi; ainsi la température dans le ballon de reflux B est-elle maintenue à 50 °C.

Le mélange à séparer, ALIMENTATION. est introduit en phase liquide dans le débutaniseur D au plateau numéro 40 à une température de 75,5 °C.

Du ballon de reflux B on soutire en phase liquide le méiange. HC, d'hydrocarbures distillés. Le débit et la composition de ce mélange HC sont indiqués dans le tableau 1. La fraction liquide résiduelle obtenue au niveau du ballon B, de même composition que le mélange HC, est envoyée en reflux en tête du débutaniseur D sous un débit de 3300 g.h$^{-1}$ ce qui représente un taux de reflux massique par rapport à l'ALIMENTATION de 0,55.

En fond du débutaniseur on récupère en phase liquide l'ETBE purifié, ETHER. Le débit et la composition du produit ETHER ainsi obtenu sont donnés dans le tableau 1.

Au plateau numéro 54 du débutaniseur D on soutire une phase liquide, SOUT, à une température de 118,4 °C. Le débit et la composition de cette phase liquide sont donnés dans le tableau 1.

Cette phase liquide est ensuite refroidie jusqu'à une température de 90 °C dans un condenseur à eau froide E2. Elle est ensuite amenée vers l'unité de pervaporation PV. Cette unité est constituée d'une cellule ayant une surface utile de perméation de 0,1 m$^2$. Elle est équipée d'une membrane réalisée suivant la méthode décrite à l'exemple 1.

L'unité de pervaporation PV produit un rétentat liquide RETE qui est renvoyé grâce à la pompe P2 vers le plateau numéro 58 du débutaniseur D, après avoir été porté dans l'échangeur E3 à une température de 119,0 °C. Le débit et la composition de ce rétentat RETE sont donnés dans le tableau 1.

L'unité de pervaporation PV produit un perméat en phase vapeur PERMEAT sous une pression de 20 mbar, qui est condensé dans le condenseur cryogénique E4, pompé par la pompe P3 et recueilli. Le débit et la composition de ce perméat, PERMEAT, sont donnés dans le tableau 1.

TABLEAU 1

| Bilan matière | | | | | | |
|---|---|---|---|---|---|---|
| CORPS (% en poids | ALIMENTATION | HC | SOUT | RETE | PERMEAT | ETHER |
| Hydrocarbures | 67,16 | 97,7 | 28,48 | 30,32 | 2,67 | 1,26 |
| Ethanol | 3,72 | 2,09 | 10,76 | 5,76 | 81,36 | 0,03 |
| ETBE | 28,63 | <1ppm | 55,21 | 58,75 | 5,18 | 98,60 |
| ABT | 0,30 | 29 ppm | 4,58 | 4,20 | 9,91 | 0,05 |
| DEE | 0,05 | 64 ppm | 0,97 | 0,97 | 0,88 | 0,06 |
| Eau | 0,14 | 0,20 | <1 ppm | <1 ppm | 3 ppm | <1 ppm |
| Débit g.h-1 | 6000,0 | 4097,0 | 2547,0 | 2378,0 | 169,0 | 1734,0 |

Cet exemple illustre bien les avantages du procédé de l'invention, à savoir que l'ETBE peut ainsi être produit à une teneur en alcools (éthanol + ABT), totale inférieure à 0,1 % en poids, et aussi que 93 %, soit l'essentiel de l'ABT produit par la section réactionnelle, se retrouve dans le PERMEAT et peut donc être recyclé vers ladite section réactionnelle, limitant ainsi la conversion de l'isobutène en ABT. Il faut également noter que la quantité d'ETBE recueillie dans le PERMEAT et pouvant donc être recyclée vers la section réactionnelle est négligeable puisqu'elle ne représente que 0,51 % de l'ETBE produit. Le recyclage d'une quantité aussi minime d'ETBE vers la section réactionnelle n'est pas susceptible de perturber le fonctionnement de ladite section.

**Revendications**

1. Procédé de séparation d'un mélange d'éthyl tertiobutyl éther (ETBE) et d'éthanol renfermant en outre notamment des hydrocarbures en C$_4$, ledit procédé étant caractérisé en ce qu'il comprend les étapes suivantes :

(a) on introduit le mélange à séparer dans une zone de distillation dite zone de débutanisation, d'où on recueille en tête la quasi-totalité des hydrocarbures en C$_4$ et on recueille en fond l'ETBE purifié ;
(b) on soutire latéralement de ladite zone de distillation au moins une phase que l'on envoie dans une zone de perméation d'où il sort un rétentat qui est recyclé vers la zone de distillation et un perméat comprenant principalement de l'éthanol, ladite zone de perméation étant équipée d'une membrane de séparation comprenant au moins une couche sélective constituée d'un film dense d'un polymère formé à partir d'au moins un monomère de diméthyl amino éthyl méthacrylate (DMAEMA).

**2.** Procédé selon la revendication 1, caractérisé en ce que ledit polymère est choisi parmi les homopolymères du DMAEMA, les copolymères formés entre le DMAEMA et le N-vinylcaprolactame ou la N-vinyl pyrrolidone et les terpolymères formés entre le DMAEMA, le N-vinylcaprolactame et la N-vinyl pyrrolidone.

**3.** Procédé selon l'une des revendications 1 et 2, caractérisé en ce que le mélange à séparer provient d'une zone de réaction d'éthérification entre l'isobutène et l'éthanol.

**4.** Procédé selon l'une des revendications 1 à 3, caractérisé en ce que le mélange ETBE/éthanol à séparer contient, outre une proportion d'hydrocarbures en $C_4$, également jusqu'à 1 % en poids d'eau, jusqu'à 1 % en poids d'alcool butylique tertiaire (ABT), jusqu'à 1 % en poids de diéthyléther (DEE), jusqu'à 1 % en poids d'éthyl 2-butyl éther (E2BE) jusqu'à 5 % en poids d'hydrocarbures en $C_5$ et jusqu'à 5 % en poids d'hydrocarbures en $C_3$.

**5.** Procédé selon la revendication 4, caractérisé en ce que, dans l'étape (a), l'effluent de tête de la zone de distillation comprend la majeure partie des hydrocarbures en $C_3$ et en $C_4$, la majeure partie de l'eau, une fraction de l'éthanol et des hydrocarbures en $C_5$ du mélange à séparer et est substantiellement exempt d'ETBE, d'E2BE, d'ABT et de DEE ; l'effluent de fond de ladite zone de distillation comprend, outre l'ETBE purifié, la majeure partie de l'E2BE, une partie du DEE, une fraction des hydrocarbures en $C_5$ et moins de 1 % en poids d'éthanol et d'ABT ; et le perméat sortant de la zone de perméation consiste essentiellement en éthanol et ABT.

**6.** Procédé selon l'une des revendications 1 à 5, caractérisé en ce que l'ETBE purifié recueilli en fond de la zone de distillation contient moins de 0,1 % en poids d'alcools (éthanol et ABT).

**7.** Procédé selon l'une des revendications 1 à 6, caractérisé en ce que la phase soutirée de la zone de distillation est une phase liquide et la perméation consiste en une pervaporation.

**8.** Procédé selon l'une des revendications 1 à 6, caractérisé en ce que la phase soutirée de la zone de distillation est une phase gazeuse ou mixte et la perméation consiste en une perméation de vapeur.

**9.** Procédé selon l'une des revendications 1 à 8, caractérisé en ce que, dans l'étape (a), la zone de distillation opère sous une pression supérieure à la pression atmosphérique, de préférence comprise entre 5 et 15 bar, à une température de fond de 70 à 200 °C, à une température de tête de 30 à 100 °C, le mélange à séparer est introduit dans ladite zone de distillation au point de bulle à la pression considérée, au plateau ayant la composition la plus voisine possible de celle dudit mélange ; et en ce que, dans l'étape (b), la phase à envoyer vers la zone de perméation est soutirée au niveau d'un plateau de ladite zone de distillation correspondant sensiblement à la concentration maximale en éthanol, elle est portée à une température de 50 à 120 °C et admise dans la zone de perméation qui produit un rétentat qui est recyclé à la zone de distillation au point de bulle à la pression considérée au plateau dont le liquide a la composition la plus voisine possible de celle dudit rétentat ; et un perméat en phase vapeur à une pression inférieure à la pression atmosphérique, de préférence comprise entre 20 et 500 mbar.

**10.** Procédé selon la revendication 9, caractérisé en ce que le rétentat de la zone de perméation est au moins partiellement vaporisé avant d'être recyclé à la zone de distillation.

**11.** Procédé selon la revendication 9, caractérisé en ce que le rétentat de la zone de perméation est partiellement vaporisé, on sépare une fraction liquide que l'on recycle à la zone de distillation à un plateau inférieur à celui auquel est effectué le soutirage latéral et une fraction vapeur que l'on recycle à la zone de distillation à un plateau supérieur au plateau de recyclage de ladite fraction liquide.

**12.** Procédé selon l'une des revendications 1 à 11, caractérisé en ce que, dans l'étape (a), l'effluent vapeur de tête de la zone de distillation est condensé et une partie du condensat est envoyé en reflux en tête de ladite zone de distillation.

**13.** Procédé de synthèse d'ETBE à partir d'éthanol et de l'isobutène contenu dans une coupe $C_4$ caractérisé en ce qu'il comprend les étapes suivantes :

(1) on mélange un flux de coupe $C_4$, un appoint d'éthanol, de l'azéotrope eau-éthanol provenant de la section de lavage du raffinat de la zone de distillation D et le perméat condensé issu de la zone de perméation, et on admet le mélange ainsi formé dans une section réactionnelle R, qui opère dans des conditions d'éthérification de l'isobutène et de l'éthanol ;

(2) on alimente une zone de distillation D au moyen de l'effluent de la zone réactionnelle R qui comprend l'ETBE produit, les hydrocarbures en C4 non réactifs ou non réagis, l'excès d'éthanol et diverses impuretés : ABT, eau, hydrocarbures en C3 et $C_5$, DEE et E2BE; de la zone D on recueille en fond l'ETBE purifié, contenant des traces de DEE et d' E2BE, en tête un effluent contenant les hydrocarbures, l'eau et une fraction de l'éthanol, on soutire latéralement de la zone D une phase, envoyée vers une zone de perméation PV équipée d'une membrane de séparation comprenant au moins une couche sélective constitué d'un film dense d'un polymère formé à partir d'au moins un monomère de diméthyl amino éthyl méthacrylate (DMAEMA);

(3) on réalise la perméation de ladite phase, produisant ainsi un rêtentat appauvri en alcools (éthanol et ABT) qui est recyclé vers la zone de distillation D, et un perméat majoritairement constitué d'alcools (éthanol et ABT) qui est recyclé vers la section réactionnelle R;

(4) on envoie l'effluent vapeur de tête de la zone de distillation D, après condensation, vers une section de lavage à l'eau LD, qui produit un effluent constitué d'hydrocarbures essentiellement exempts d'éthanol et un mélange eau-éthanol dont on sépare, par distillation, en fond de l'eau, que l'on recycle vers le lavage et, en tête, un mélange azéotropique eau/éthanol à environ 5 % en poids d'eau, que l'on recycle vers la section réactionnelle R.

**14.** Procédé selon la revendication 13, caractérisé en ce que ledit polymère est choisi parmi les homopolymères de DMAEMA, les copolymères formés entre le DMAEMA et le N-vinylcaprolactame ou la N-vinyl pyrrolidone et les terpolymères formés entre le DMAEMA, le N-vinylcaprolactame et la N-vinyl pyrrolidone.

**15.** Procédé selon l'une des revendications 13 et 14, caractérisé en ce que la zone de distillation D est remplacée par une zone de distillation catalytique ou réactive D'.

**16.** Procédé selon la revendication 15, caractérisé en ce que au moins une partie du perméat produit par la zone de perméation de l'étape (3) est recyclée vers ladite zone de distillation catalytique ou réactive D'.

**17.** Procédé selon l'une des revendications 15 et 16, caractérisé en ce que au moins une partie du mélange azéotropique eau/éthanol, produit par la zone de lavage-distillation de l'étape (4) est recyclée vers ladite zone de distillation catalytique ou réactive D'.

**Claims**

**1.** A process for the separation of a mixture of ethyl tertio-butyl ether (ETBE) and ethanol also containing $C_4$ hydrocarbons in particular, said process being characterised in that it comprises the following steps:

a) introducing the mixture to be separated into a distillation zone termed the debutanising zone, from which almost the whole of the $C_4$ hydrocarbons are recovered overhead and purified ETBE is recovered from the bottom;
b) extracting at least one phase as a side stream from said distillation zone and sending it to a permeation zone from which a retentate is recycled to the distillation zone and a permeate comprising mainly ethanol is produced, said permeation zone being provided with a separation membrane comprising at least one selective layer constituted by a dense film of a polymer formed from at least one dimethylaminoethyl methacrylate monomer (DMAEMA).

**2.** A process according to claim 1, characterised in that said polymer is selected from homopolymers of DMAEMA, copolymers of DMAEMA and N-vinylcaprolactame or N-vinyl pyrrolidone and terpolymers of DMAEMA, N-vinylcaprolactame and N-vinyl pyrrolidone.

**3.** A process according to claim 1 or clai 2, characterised in that the mixture to be separated is supplied from an etherification reaction zone between isobutene and ethanol.

**4.** A process according to any one of claims 1 to 3, characterised in that the ETBE/ethanol mixture to be separated contains, in addition to a proportion of $C_4$ hydrocarbons, up to 1% by weight of water, up to 1% by weight of tertiary

EP 0 675 099 B1

butyl alcohol (TBA), up to 1% by weight of diethyl ether (DEE), up to 1% by weight of ethyl 2-butyl ether (E2BE), up to 5% by weight of $C_5$ hydrocarbons and up to 5% by weight of $C_3$ hydrocarbons.

5. A process according to claim 4, characterised in that, in step a), the overhead effluent from the distillation zone comprises the major portion of the $C_3$ and $C_4$ hydrocarbons, the major portion of the water, a fraction of the ethanol and the $C_5$ hydrocarbons from the mixture to be separated and is substantially free of ETBE, E2BE, TBA and DEE; and the bottom effluent from said distillation zone comprises, in addition to purified ETBE, the major portion of the E2BE, a portion of the DEE, a fraction of the $C_5$ hydrocarbons and less than 1% by weight of ethanol and TBA; and the permeate leaving the permeation zone consists mainly of ethanol and TBA.

6. A process according to any one of claims 1 to 5, characterised in that the purified ETBE recovered from the bottom of the distillation zone contains less than 0.1% by weight of alcohols (ethanol and TBA).

7. A process according to any one of claims 1 to 6, characterised in that the phase extracted from the distillation zone is a liquid phase and the permeation consists of pervaporation.

8. A process according to any one of claims 1 to 6, characterised in that the phase extracted from the distillation zone is a gaseous or mixed phase and the permeation consists of vapour permeation.

9. A process according to any one of claims 1 to 8, characterised in that, in step a), the distillation zone operates at a pressure which is greater than atmospheric pressure, preferably between 5 and 15 bar, at a bottom temperature of 70°C to 200°C, at a top temperature of 30°C to 100°C, the mixture to be separated is introduced into said distillation zone at the bubble point at the appropriate pressure, to the tray with the closest composition possible to that of said mixture; and in that, in step b), the phase to be sent to the permeation zone is extracted from a tray in said distillation zone which substantially corresponds to the maximum concentration of the ethanol, it is heated to a temperature of 50°C to 120°C and introduced into the permeation zone which produces a retentate which is recycled to the distillation zone at the bubble point at the appropriate pressure to the tray at which the liquid has the closest possible composition to that of said retentate; and a vapour phase permeate is produced at a pressure which is lower than atmospheric pressure preferably between 20 and 500 mbar.

10. A process according to claim 9, characterised in that the retentate from the permeation zone is at least partially vaporised before being recycled to the distillation zone.

11. A process according to claim 9, characterised in that the retentate from the permeation zone is partially vaporised, a liquid fraction is separated and recycled to the distillation zone to a lower tray than that from which the side stream is extracted and a vapour fraction is separated which is recycled to the distillation zone to a higher tray than the recycle tray for said liquid fraction.

12. A process according to any one of claims 1 to 11, characterised in that, in step a), the overhead vapour effluent from the distillation zone is condensed and a portion of the condensate is sent as a reflux to the top of the distillation zone.

13. A process for the synthesis of ETBE from ethanol and from isobutene contained in a $C_4$ cut, characterised in that it comprises the following steps:

1) mixing a $C_4$ cut, ethanol, a water - ethanol azeotrope from the washing section for the raffinate from distillation zone D and the condensed permeate from the permeation zone, and introducing the mixture formed into a reaction section R, which operates under conditions for etherification of isobutene and ethanol ;
2) supplying a distillation zone D with effluent from reaction zone R which comprises the ETBE product, non reactive or unreacted $C_4$ hydrocarbons, excess ethanol and various impurities : TBA, water, $C_3$ and $C_5$ hydrocarbons, DEE and E2BE ; recovering from the bottom of zone D, purified ETBE which contains traces of DEE and E2BE, and recovering overhead an effluent containing hydrocarbons, water and a fraction of the ethanol, and extracting a phase from zone D as a side stream and sending it to a permeation zone PV provided with a separation membrane comprising at least one selective layer constituted by a dense film of a polymer formed from at least one dimethylaminoethyl methacrylate monomer (DMAEMA) ;
3) carrying out permeation on said phase, producing a retentate which is depleted in alcohol (ethanol and TBA) which is recycled to distillation zone D, and a permeate which is mainly constitued by alcohols (ethanol and TBA) which is recycled to reaction zone R ;

12

4) sending the overhead effluent vapour from distillation zone D, following condensation, to a water washing section LD, which produces an effluent constituted by hydrocarbons which are substantially free of ethanol and a water - ethanol mixture which is separated by distillation into a water bottom product which is recycled to the washing section and an overhead azeotropic water/ethanol mixture containing about 5% by weight of water, which is recycled to reaction section R.

14. A process according to claim 13, characterised in that said polymer is selected from homopolymers of DMAEMA, copolymers of DMAEMA and N-vinylcaprolactame or N-vinyl pyrrolidone and terpolymers of DMAEMA, N-vinylcaprolactame and N-vinyl pyrrolidone.

15. A press according to any one of claims 13 and 14, characterised in that distillation zone D is replaced by a catalytic or reactive distillation zone D'.

16. A process according to claim 15, characterised in that at least a portion of the permeate produced by the permeation zone of step 3) is recycled to said catalytic or reactive distillation zone D'.

17. A process according any one of claims 15 and 16, characterised in that at least a portion of the azeotropic water/ethanol mixture produced by the washing - distillation zone in step 4) is recycled to said catalytic or reactive distillation zone D'.

## Patentansprüche

1. Verfahren zum Trennen eines Gemisches aus Ethyl-tert-butyl-ether (ETBE) und Ethanol, u.a. insbesondere $C_4$ Kohlenwasserstoffe enthaltend, wobei das Verfahren sich dadurch auszeichnet, daß es die folgenden Stufen umfaßt:

   (a) man führt das zu trennende Gemisch in eine Destillationszone, eine sog. Debutanisierungszone, ein, aus der man am Kopf fast die Gesamtheit der $C_4$ Kohlenwasserstoffe und am Boden das gereinigte ETBE sammelt:

   (b) man zieht seitlich aus der Destillationszone wenigstens eine Phase ab, die man in eine Permeationszone schickt, aus der ein Retentat, welches gegen die Destillationszone rezykliert wird, sowie ein Permeat, das hauptsächlich Ethanol enthält, austritt, wobei diese Permeationszone mit einer Trennmembran ausgestattet ist, die wenigstens eine selektive Schicht enthält, die aus einem dichten Film eines Polymers aufgebaut ist, das aus wenigstens einem Dimethylaminoethylmethacrylatmonomer (DMAEMA) gebildet ist.

2. Verfahren nach Anspruch 1, dadurch gekennzeichnet, daß dieses Polymer gewählt ist aus den Homopolymeren des DMAEMA, den Copolymeren, die zwischen dem DMAEMA und dem N-Vinylcaprolactam oder dem N-Vinylpyrrolidon gebildet sind sowie den Terpolymeren, die zwischen dem DMAEMA, dem N-Vinylcaprolactam und dem N-Vinylpyrrolidon gebildet sind.

3. Verfahren nach einem der Ansprüche 1 und 2, dadurch gekennzeichnet, daß das zu trennende Gemisch aus einer Reaktionszone der Etherifizierung zwischen Isobuten und Ethanol stammt.

4. Verfahren nach einem der Ansprüche 1 bis 3, dadurch gekennzeichnet, daß das zu trennende ETBE/Ethanolgemisch außer einem Anteil von $C_4$ Kohlenwasserstoffen auch bis zu 1 Gew.-% Wasser, bis zu 1 Gew.-% tertiären Butylalkohol (ABT), bis zu 1 Gew.-% Diethylether (DEE), bis zu 1 Gew.-% Ethyl-2-butylether (E2BE), bis zu 5 Gew.-% $C_5$ Kohlenwasserstoffe und bis zu 5 Gew.-% $C_3$ Kohlenwasserstoffe enthält.

5. Verfahren nach Anspruch 4, dadurch gekennzeichnet, daß in der Stufe (a) der Kopfabstrom der Destillationszone den größeren Teil der $C_3$ und $C_4$ Kohlenwasserstoffe, den größeren Teil des Wassers, eine Fraktion von Ethanol und der $C_5$ Kohlenwasserstoffe des zu trennenden Gemisches umfaßt und im wesentlichen frei von ETBE, E2BE, ABT und DEE ist; der Bodenabstrom dieser Destillationszone außer dem gereinigten ETBE, den größeren Teil des E2BE, einen Teil des DEE, eine Fraktion $C_5$ der Kohlenwasserstoffe und wenigstens 1 Gew.-% Ethanol und ABT umfaßt; und das aus der Permeationszone austretende Permeat im wesentlichen aus Ethanol und ABT besteht.

6. Verfahren nach einem der Ansprüche 1 bis 5, dadurch gekennzeichnet, daß das gereinigte am Boden der Destillationszone gesammelte ETBE wenigstens 0,1 Gew.-% Alkohole (Ethanol und ABT) enthält.

7. Verfahren nach einem der Ansprüche 1 bis 6, dadurch gekennzeichnet, daß die aus der Destillationszone abgezogene Phase eine flüssige Phase ist und die Permeation aus einer Perverdampfung besteht.

8. Verfahren nach einem der Ansprüche 1 bis 6, dadurch gekennzeichnet, daß die aus der Destillationszone abgezogene Phase eine gasförmige oder gemischte Phase ist und die Permeation aus einer Wasserdampfpermeation besteht.

9. Verfahren nach einem der Ansprüche 1 bis 8, dadurch gekennzeichnet, daß in der Stufe (a) die Destillationszone unter einem Druck höher als dem atmosphärischen Druck, bevorzugt zwischen 5 und 15 bar, bei einer Bodentemperatur von 70 bis 200°C, bei einer Kopftemperatur von 30 bis 100°C betrieben wird, das zu trennende Gemisch in diese Destillationszone beim Siedepunkt beim betrachteten Druck auf dem Boden, der die Zusammensetzung hat, die diesem Gemisch am weitesten benachbart ist, eingeführt wird; und daß in der Stufe (b) die gegen die Permeationszone zu schickende Phase auf dem Niveau eines Bodens dieser Destillationszone abgezogen wird, der im wesentlichen der maximalen Ethanolkonzentration entspricht, daß sie auf eine Temperatur von 50 bis 120°C gebracht und in die Permeationszone eingelassen wird, die ein Retentat, das zur Destillationszone beim Siedepunkt beim betrachteten Druck auf den Boden rezykliert wird, dessen Flüssigkeit, die die dem Retentat am weitesten benachbarte Zusammensetzung hat; und ein Permeat in Dampfphase bei einem Druck kleiner als dem atmosphärischen Druck, bevorzugt zwischen 200 und 500 mbar, erzeugt.

10. Verfahren nach Anspruch 9, dadurch gekennzeichnet, daß das Retentat aus der Permeationszone wenigstens teilweise verdampft wird, bevor es in die Destillationszone rezykliert wird.

11. Verfahren nach Anspruch 9, dadurch gekennzeichnet, daß das Retentat aus der Permeationszone teilweise verdampft wird, daß man eine flüssige Fraktion, die man zur Destillationszone auf einen Boden niedriger als dem rezykliert, auf dem das seitliche Abziehen vorgenommen wird und eine Dampffraktion trennt, die man zur Destillationszone auf einen Boden oberhalb des Rezyklierungsbodens dieser flüssigen Fraktion rezykliert.

12. Verfahren nach einem der Ansprüche 1 bis 11, dadurch gekennzeichnet, daß in der Stufe (a) der Dampfkopfabstrom der Destillationszone kondensiert wird und ein Teil des Kondensats im Rückstrom zum Kopf dieser Destillationszone geschickt wird.

13. Verfahren zur Synthese von ETBE aus Ethanol und in einem $C_4$ Schnitt enthaltenden Isobuten, dadurch gekennzeichnet, daß es die folgenden Stufen umfaßt:

(1) man mischt einen Strom von der Fraktion $C_4$, einen Ethanolzusatz, das aus dem Waschabschnitt des Raffinats der Destillationszone D stammende Wasser Ethanolazeotrop und das kondensierte Permeat, das aus der Permeationszone stammt und man gibt das so gebildete Gemisch in einen Reaktionsabschnitt R, der unter Etherifizierungsbedingungen des Isobutens und des Ethanols arbeitet;

(2) man speist eine Destillationszone D vermittels des Abstroms aus der Reaktionszone R, der das erzeugte ETBE, die nicht reaktiven oder nicht reagiert habenden $C_4$ Kohlenwasserstoffe, den Überschuß an Ethanol und verschiedene Verunreinigungen: ABT, Wasser, $C_3$ und $C_5$ Kohlenwasserstoffe, DEE und E2BE umfaßt; aus der Zone D sammelt man am Boden das gereinigte ETBE, welches Spuren von DEE und E2BE enthält, am Kopf einen die Kohlenwasserstoffe, Wasser und eine Ethanolfraktion umfassenden Abstrom; seitlich zieht man aus der Zone D eine Phase ab, die zu einer Permeationszone PV geschickt wurde, welche mit einer Trennmembran ausgestattet ist, die wenigstens eine selektive Schicht umfaßt, die aus einem dichten Film eines Polymers gebildet ist, das aus wenigstens einem Monomer von Dimethylaminoethylmethacrylat (DMAEMA) gebildet ist;

(3) man realisiert die Permeation dieser Phase und erzeugt so ein an Alkoholen (Ethanol und ABT) verarmtes Retentat, das zur Destillationszone D zurückgeschickt wird, sowie ein Permeat, das überwiegend aus Alkoholen (Ethanol und ABT) gebildet wird, welches zum Reaktionsabschnitt R zurückgeschickt wird;

(4) man schickt den Kopfdampfabstrom der Destillationszone D nach Kondensation zu einem Abschnitt zum Waschen mit Wasser LD, der einen Abstrom erzeugt, der im wesentlichen aus Ethanol freien Kohlenwasserstoffen und einem Wasser-Ethanolgemisch gebildet ist, von dem man durch Destillation am Boden Wasser abtrennt, das man zum Waschen rezykliert und am Kopf ein azeotropisches Wasser/Ethanolgemisch mit etwa 5 Gew.-% Wasser, das man zum Reaktionsabschnitt R rezykliert.

14. Verfahren nach Anspruch 13, dadurch gekennzeichnet, daß dieses Polymer gewählt ist aus den Homopolymeren von DMAEMA, den Copolymeren, die zwischen dem DMAEMA und dem N-Vinylcaprolactam oder dem N-Vinylpyrrolidon gebildet ist und den Terpolymeren, die zwischen dem DMAEMA, dem N-Vinylcaprolactam und dem N-Vinylpyrrolidon gebildet sind.

15. Verfahren nach einem der Ansprüche 13 und 14, dadurch gekennzeichnet, daß die Destillationszone D ersetzt wird durch eine katalytische oder reaktive Zone D'.

16. Verfahren nach Anspruch 15, dadurch gekennzeichnet, daß wenigstens ein Teil des durch die Permeationszone der Stufe (3) erzeugten Permeats zu dieser katalytischen oder reaktiven Destillationszone D' rezykliert wird.

17. Verfahren nach einem der Ansprüche 15 und 16, dadurch gekennzeichnet, daß wenigstens ein Teil des azeotropen durch die Wasch/Destillationszone der Stufe (4) erzeugten Wasser/Ethanolgemisches gegen diese katalytische oder reaktive Destillationszone D' rezykliert wird.

**FIG.1**